# FASCICULE DE BREVET EUROPEEN

(11) **EP 2 440 218 B1**
(45) Date de publication et mention de la délivrance du brevet: **05.08.2015**
(21) Numéro de dépôt: 10728357.4
(22) Date de dépôt: 11.06.2010
(51) Int. Cl.: A61K 35/76

(54) **SOUCHE ATTENUEE DE MYXOMA VIRUS POUR L'UTILISATION COMME MÉDICAMENT ONCOLYTIQUE**
EIN ATTENUIERTER STAMM VON MYXOMA VIRUS ALS ONKOLYTISCHER HEILMITTEL
AN ATTENUATED STRAIN OF MYXOMA VIRUS FOR USE AS AN ONCOLYTIC MEDICAMENT

(30) Priorité: 11.06.2009 FR 0902835
(43) Date de publication de la demande: 18.04.2012
(73) Titulaire: Institut National de la Recherche Agronomique (INRA), 75007 Paris (FR); Ecole Nationale Veterinaire Toulouse, 31300 Toulouse (FR)
(72) Inventeur: BERTAGNOLI, Stéphane, F-31410 Lavernose-lacasse (FR); GRETILLAT, Magalie, 69100 Villeurbanne (FR); GELFI, Jacqueline, F-31170 Tournefeuille (FR); CAMUS-BOUCLAINVILLE, Christelle, 31100 Toulouse (FR)
(74) Mandataire: Marcadé, Véronique
(86) Numéro de dépôt international: PCT/IB2010/052605
(87) Numéro de publication internationale: WO 2010/143160

(56) Documents cités:
- WO-A-2007/143548
- FR-A- 2 736 358
- LABUDOVIC A. ET AL.: "Sequence mapping of the Californian MSW strain of myxoma virus." ARCH. VIROL., vol. 149, 2004, pages 553-570, XP002600083

## Description

La présente invention est relative à une souche atténuée vaccinale de *Myxoma virus* pour l'utilisation comme agent oncolytique.

Un virus oncolytique est un virus qui lyse préférentiellement les cellules cancéreuses, tout en n'ayant que peu d'effet sur les cellules non cancéreuses. La virothérapie oncolytique constitue une approche thérapeutique intéressante pour compléter l'arsenal des thérapies anticancéreuses disponibles.

Le virus de la myxomatose (MYXV) également dénommé *Myxoma virus,* appartient au genre *Leporipoxvirus* dans la famille des *Poxviridae.* Il est l'agent responsable de la myxomatose, une maladie infectieuse majeure chez le lapin européen, et endémique en Europe. Ce virus présente un spectre d'hôte étroit et n'est pas pathogène pour l'Homme.

La séquence complète du génome de la souche Lausanne du MYXV (souche pathogène de référence) est connue (CAMERON et al., Virology, 264, 298-318, 1999; GenBank NC_001132). Ce génome est constitué d'une molécule linéaire d'ADN bicaténaire d'environ 162 kpb. Il contient une région centrale fortement conservée dans la famille des *Poxviridae,* contenant les gènes essentiels à la réplication virale, encadrée par des zones plus variables contenant des génes dits de virulence, et deux répétitions inversées terminales (RIT) de 11,5 kbp. Il comprend au total 170 cadres de lecture ouverts dont 12 sont présents en double copie dans les RIT. Certaines des protéines codées par ces 12 cadres de lecture ont été identifiées comme constituant des facteurs de virulence, interférant avec les défenses immunitaires de l'hôte. On citera notamment la serpine Serp1, décrite comme possédant des propriétés inhibitrices de la réponse inflammatoire et de l'activation du complément ; les virocepteurs M-T1, M-T2, et M-T7, décrits comme interférant avec des chimiokines impliquées dans la réponse antivirale: CC-chimiokines dans le cas de M-T1, TNF dans le cas de M-T2, et l'IFN-gamma dans celui de M-T7 ; les protéines M-T2, MT4, et MT5, qui constitueraient des inhibiteurs de l'apoptose des cellules infectées.

Malgré sa grande spécificité d'hôte, il a été montré que le MYXV pouvait infecter de façon productive un grande variété de cellules tumorales *in vitro* (SYPULA et al., Gen Ther Mol Biol, 8, 103-114, 2004), et qu'il présentait une activité oncolytique dans les cellules d'adénocarcinome pancréatique humain *in vitro* (WOO et al., Ann Surg Oncol, 15, 2329-35, 2008), et dans des modèles murins de gliomes, de médulloblastomes et de tumeurs rhabdoïdes humaines *in vivo* Lun (LUN et al., Cancer Res, 65, 9982-90, 2005; LUN et al., Cancer Res, 67, 8818-27, 2007 ; WU et al., Clin Cancer Res, 14, 1218-27, 2008). Il a ainsi été proposé de l'utiliser comme agent oncolytique (Demande PCT WO2004/078206). La Demande PCT WO2007/143548, et BARRETT et al. (J. Neurovirol, 13, 549-560, 2007), décrivent des essais d'inactivation de certains gènes de *Myxoma virus* codant pour des protéines impliquées dans le tropisme cellulaire vis-à-vis des cellules de lapin (M11L, M063, M135, M136, M-T2, M-T4, M-T5 et M-T7), dans le but de tester les effets de cette inactivation sur la capacité des *Myxoma virus* à infecter et tuer des cellules de gliome humain, et d'utiliser ces *Myxoma virus* pour le traitement du cancer, notamment du gliome ; les *Myxoma virus* dans lesquels le gène M063 ou le gène M135 a été inactivé se répliquent dans les cellules de gliome plus efficacement que le virus de type sauvage, et réduisent la viabilité des cellules de gliome qu'ils ont infectées.

Il a été rapporté que la permissivité des cellules tumorales humaines à l'infection par un *Myxoma virus* était liée d'une part au niveau d'activation de la sérine/thréonine kinase Akt-1/PKB dans la cellule tumorale, et d'autre part à l'expression chez le virus d'une protéine M-T5 fonctionnelle, qui est capable d'induire la phosphorylation et l'activation d'Akt-1/PKB. On peut ainsi distinguer 3 types de cellules tumorales : chez les cellules de type I, Akt-1/PKB est fortement activée de manière constitutive, et ces cellules sont permissives à l'infection par un *Myxoma virus* même si celui-ci n'exprime pas M-T5 ; chez les cellules de type II, le niveau basal d'activation Akt-1/PKB est faible, mais elle est activée par l'interaction avec M-T5 : ces cellules ne sont permissives qu'aux *Myxoma virus* exprimant M-T5 ; enfin, chez les cellules de type III, le niveau basal d'activation Akt-1/PKB est également faible, et elle n'est pas activée par M-T5 : ces cellules ne sont pas permissives aux *Myxoma virus*. Le traitement de cellules tumorales non permissives avec un agent capable d'augmenter l'activation d'Akt-1/PKB, tel que la rapamycine, permet d'améliorer grandement leur sensibilité à l'infection par les *Myxoma virus* (STANFORD & MCFADDEN, Expert Opin Biol Ther, 7, 1415-25, 2007 ; LUN et al., Cancer Res, 67, 8818-27, 2007).

Les Inventeurs ont maintenant constaté qu'une souche atténuée vaccinale de *Myxoma virus*, la souche SG33, se répliquait plus efficacement et diffusait mieux dans les cellules tumorales, et possédait une activité oncolytique supérieure et un spectre d'action plus large qu'une souche sauvage telle que la souche Lausanne.

La souche SG33 (CNCM I-1594) est décrite dans le Brevet FR 2736358. Les Inventeurs ont comparé le génome de SG33 avec celui de la souche de référence Lausanne, afin de déterminer quelles étaient les différences entre ces deux souches pouvant affecter la production ou la fonction de protéines virales.

Ils ont constaté que la souche SG33 contient une délétion d'environ 15 kb dans la partie droite de son génome. Les gènes affectés par cette délétion sont listés dans le tableau I ci-après :

**Tableau I**

| Nom du gène | Position sur le génome^{a} | Produit (numéro d'accès GenBank)^{a} | Fonction putative/homologues connus |
|---|---|---|---|
| M151R | 146684-147685 | Serp2 (NP_051864) | Serpine/SPI 2 du virus de la vaccine |
| M152R | 147688-148488 | Serp3 (NP_051865) | Serpine |
| M153R | 148526-149146 | M153R/MV-Lap (NP_051866) | Scrapine |
| M154L | 149883-149239 | gp120-like (NP_051868) | Fonction inconnue/M2 du virus de la vaccine |
| M156R | 149997-150305 | M156R(NP_051869) | Résistance aux IFNs/K3L du virus de la vaccine ; elF2 alpha |
| M008.1R | 150313-151422 | Serp1 (NP_051870) | Serpine |
| M008R | 151400-152947 | M-T8 (NP_051871) | Protéine à motif Kelch/A55R du virus de la vaccine |
| M007R | 152998-153789 | M-T7(NP_051872) | Récepteur IFN-gamma/B8R du virus de la vaccine |
| M006R | 153826-155355 | M-T6 (NP_051873) | Protéine à motif Kelch/A55R du virus de la vaccine |
| M005R | 155391-156842 | M-T5 (NP_051874) | Protéine à motif ankyrine/B4R du virus de la vaccine |
| M004.1 R | 156862-157134 | M4.1 (NP_051875) | Fonction inconnue/C2L du virus swinepox |
| M004R | 157138-157851 | M-T4 (NP_051876) | Facteur de virulence/B9R du virus de la vaccine |
| M003.2R | 158084-158425 | M3.2 (NP_051877) | Fonction inconnue/T3C des capripoxvirus |
| M003.1R | 158495-158950 | m3.1L/R (NP_051878) | Fonction inconnue/B15R du virus de la vaccine |
| M002R | 159129-160109 | M-T2(NP_051879) | Récepteur soluble du TNF alpha/B28R du virus de la vaccine |
| M001R | 160189-160971 | M-T1 (NP_051880) | Protéine liant les chimiokines/B29R du virus de la vaccine |

| | | | |
|---|---|---|---|
| ^{a}Les positions sur le génome et les numéros d'accès GenBank sont ceux des gènes et protéines correspondants chez la souche de référence Lausanne. | | | |

Les gènes M151R et M001R ne sont que partiellement délétés, donnant lieu à des protéines tronquées inactives. Les gènes M152R, M153R, M154L, M156R, ainsi que les gènes de l'ITR droite M008.1R, M008R, M007R, M006R, M005R, M004.1R, M004R, M003.2R, M003.1R, et M002R sont complètement délétés. Les protéines codées par les gènes M152R, M153R, M154L, M156R, sont donc absentes ; en revanche, une autre copie des gènes M008.1R, M008R, M007R, M006R, M005R, M004.1R, M004R, M003.2R, M003.1R, et M002R étant présente dans la RIT gauche, on peut supposer que les protéines correspondantes sont toujours produites, bien que probablement en quantité plus faible.

Une autre différence entre le génome de la souche SG33 et celui de la souche Lausanne se situe au niveau du gène M011L (positions 14125-13628 dans le génome de la souche Lausanne), codant pour un inhibiteur de l'apoptose (M11L, GenBank NP_051725).

Dans la souche SG33 ce gène est interrompu par un codon stop, conduisant à l'apparition de deux nouveaux cadres de lecture potentiels et à la production de deux protéines tronquées, M11aL (correspondant aux 32 premiers acides aminés de M11L) et M11bL (débutant par la méthionine en position 52 de M11L). La protéine M11aL est probablement inactive, et il est possible que certaines des fonctions de M11L soient altérées chez M11bL.

La présente invention a pour objet le *Myxoma, virus* atténué SG33 (CNCM I-1594), pour l'utilisation comme médicament oncolytique dans le traitement d'une tumeur dont les cellules sont sensibles à la lyse par ledit *Myxoma virus.*

Avantageusement, pour la mise en oeuvre de la présente invention, ledit *Myxoma virus* atténué peut être modifié pour exprimer un gène d'intérêt (par exemple un gene thérapeutique de type *Thymidine kinase* d'herpes virus, ou *FCU1,* produit de la fusion entre les gènes codant pour la Cytosine désaminase et l'Uracile phosphoribosyltransférase) (ERBS et al, Cancer Gene Therapy, 15, 18-28, 2008). Des *Myxoma virus* atténués modifiés pour exprimer un gène d'intérêt sont par exemple décrits dans le Brevet FR 2736358.

Le *Myxoma virus* atténué SG33 peut être utilisé dans le traitement de tout type de cancer dont les cellules permettent leur réplication, ce qui peut aisément être déterminé en utilisant les méthodes décrites dans les Exemples ci-après. Son utilisation est particulièrement avantageuse dans le cadre du traitement de cancers colorectaux, pancréatiques et ovariens. Il peut également être utilisé dans le cadre du traitement dé tumeurs gliales, par exemple les glioblastomes.

Le *Myxoma virus* SG33 utilisé conformément à la présente invention peut être administré localement, par injection intra-tumorale. Il peut également être administré par voie générale, notamment intra-veineuse; en effet, le fait qu'il présente une meilleure diffusion au sein de cultures de cellules tumorales *in vitro* et une meilleure efficacité oncolytique à faible multiplicité d'infection que le *Myxoma virus* sauvage, peut permettre de limiter les conséquences de la perte de particules virales avant l'atteinte de la zone tumorale cible.

La présente invention sera mieux comprise à l'aide du complément de description qui va suivre, qui se réfère à des exemples non-limitatifs illustrant les propriétés du *Myxoma virus* SG33, comparées à celles d'une souche sauvage de *Myxoma virus.*

### EXEMPLE 1: COMPARAISON DE L'EFFICACITE DE REPLICATION DE LA SOUCHE SG33 ET DE LA SOUCHE SAUVAGE TOULOUSE 1 EN CELLULES TUMORALES HUMAINES.

La souche SG33 est une souche de *Myxoma virus* atténuée par passages en série sur culture cellulaire; la souche TOULOUSE 1 est une souche virulente classique de *Myxoma virus,* équivalente à la souche de référence LAUSANNE. Les souches SG33 et TOULOUSE 1 sont décrites dans le Brevet FR 2736358, et ont été respectivement déposées auprès de la Collection Nationale de Cultures de Micro-organismes (C.N.C.M.) sous les numéros I-1594 et 1-1592.

Les lignées de cellules tumorales humaines utilisées sont listées dans le Tableau II ci-dessous.

**Tableau II**

| Lignée cellulaire | Collection de reference (dénomination) | Type cellulaire | Niveau basal d'activation dAkt | Référence (activation d'Akt) |
|---|---|---|---|---|
| RK-13 | ATCC (CCL-37) | Cellules de rein de lapin | - | - |
| HUV-EC-C | ATCC (CRL-1730) | Cellules endothéliales humaines normales | - | - |
| HCT-116 | ATCC (CCL-247) | Adénocarcinome colorectal humain | élevé | 1, 2 |
| Caco-2 | ATCC (HTB-37) | Adénocarcinome colorectal humain | élevé | 2 |
| PANC-1 | ATCC (CRL-1469) | Adénocarcinome pancréatique humain | élevé | 3, 4 |
| Capan-2 | ATCC (HTB-80) | Adénocarcinome pancréatique humain | élevé | 3, 4 |
| SK-OV-3 | ATCC (HTB-77) | Adénocarcinome ovarien humain | élevé | 5 |
| OV-CAR-5 | NCI60 (OV-CAR-5) | Adénocarcinome ovarien humain | faible | 5 |
| ATCC: American type Culture Collection | | | | |
| NCI60: Collection de 60 lignées cellulaires tumorales de l'Institut national du cancer des États-Unis | | | | |
| (1) WANG et al., PNAS, 103, 4640-5, 2006; (2) SHIMIZU et al, Clin Cancer Res. 11, 2735-46, 2005. ; (3) BONDAR et al, Mol Cancer Therap, 12, 989-97, 2002; (4) WOO et al, Ann Surg Oncol, 15, 2329-35, 2008 ; (5) ALTOMARE et al, Oncogene. 23. 5853-7, 2004. | | | | |

Chaque lignée cellulaire (1,2x10⁶ cellules) a été infectée par les souches Toulouse 1 ou SG33 à une M.O.I. (multiplicité d'infection) de 3 (adsorption 90 minutes à 4°C, puis incubation à 37°C, en atmosphère à 5% de CO₂) puis la production virale a été mesurée 4, 12, 24 et 48 heures post-infection. Pour cela, à chaque point de cinétique, les cellules ont été congelées, puis ont subi trois cycles de congélation-décongélation, et le surnageant appliqué sur cellules RK13 pour titrage par la méthode des plages. L'efficacité de réplication est déterminée par le rapport entre le titre viral initial, immédiatement après l'adsorption à 4°C (T0) et le titre viral 48 heures post-infection (T48).

Les résultats (rapport T48/T0) obtenus à partir des courbes de titrage (réalisées en triplicat) sont représentés dans le Tableau III ci-dessous.

**Tableau III**

| Lignée cellulaire | Infection Toulouse 1 | Infection SG33 |
|---|---|---|
| RK-13 | 54,81 | 92,59 |
| HUV-EC-C | 0.22 | 2 |
| HCT-116 | 1,15 | 17,13 |
| Caco-2 | 2,71 | 30,36 |
| PANC-1 | 3,67 | 4,33 |
| Capan-2 | 3,96 | 5,28 |
| SK-OV-3 | 3, 34 | 5,71 |
| OV-CAR-5 | 4,71 | 23,36 |

Les résultats obtenus à partir des courbes de titrage (réalisées en triplicat) montrent que la souche SG33 possède une capacité multiplicative supérieure, se manifestant par un rendement significativement meilleur.

Des expériences d'adsorption ont en outre été effectuées sur les lignées cellulaires RK-13, HCT-116, Caco-2, PANC-1, Capan-2, SK-OV-3, et OV-CAR-5. 10⁶ cellules ont été infectées à M.O.I. de 5 par l'un ou l'autre des virus (Toulouse 1 ou SG33) à 4°C, puis lavées deux fois avant d'être congelées et de subir trois cycles de congélation-décongélation. Les virus adsorbés sont ensuite titrés sur cellules RK13.

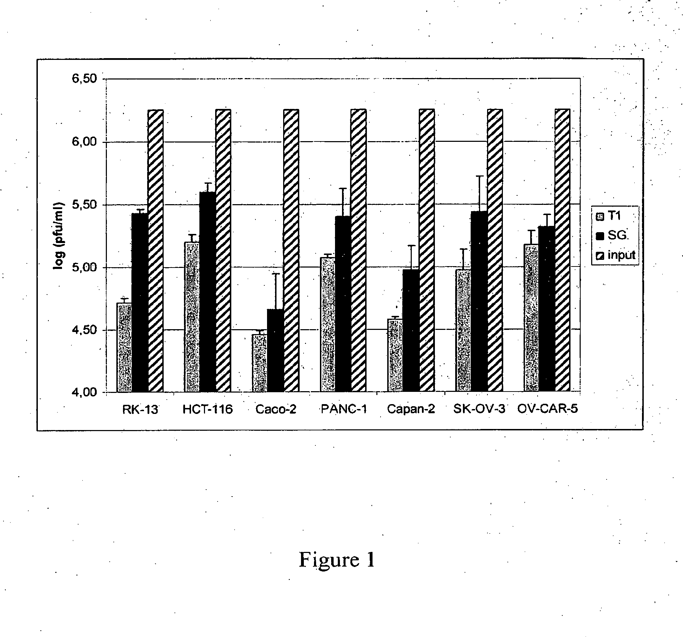

Les résultats représentant la moyenne de trois expériences indépendantes, sont illustrés par la Figure 1. En abscisse: souches testées; en ordonnée : titre viral ; Tl= Toulouse 1 ; SG= SG33 ; input= quantité théorique de virus dans chaque inoculum.

Ces résultats montrent que la souche SG33 s'adsorbe plus efficacement que la souche Toulouse 1 sur la totalité des lignées cellulaires testées.

### EXEMPLE 2 : COMPARAISON DES CINETIQUES DE DIFFUSION DES VIRUS TOULOUSE 1 ET SG33 EXPRIMANT LA BETA-GALACTOSIDASE.

Afin de suivre la diffusion virale au sein des tapis cellulaires infectés, des virus Toulouse 1 et SG33 exprimant la Béta-galactosidase, après insertion du gène Lac Z, sous la dépendance d'un promoteur poxviral, dans le locus TK des genomes viraux (BERTAGNOLI et al., 1996, J. Virol., 70(8):5061-6) ont été utilisés.

Chaque lignée cellulaire (lignées tumorales HCT-116, PANC-1, et SK-OV-3, et lignées témoins HUV-EC-C et RK13), à 90-95 % de confluence, a été infectée à une M.O.I. de 0,00005 et le nombre de foyers viraux (colorés en bleu) par cupule (10⁶ cellules) a été compté à 24, 48, 72, et 96 heures post-infection (réalisé en triplicat), après fixation des cupules correspondantes et application d'une couche d'agar contenant du X-Gal.

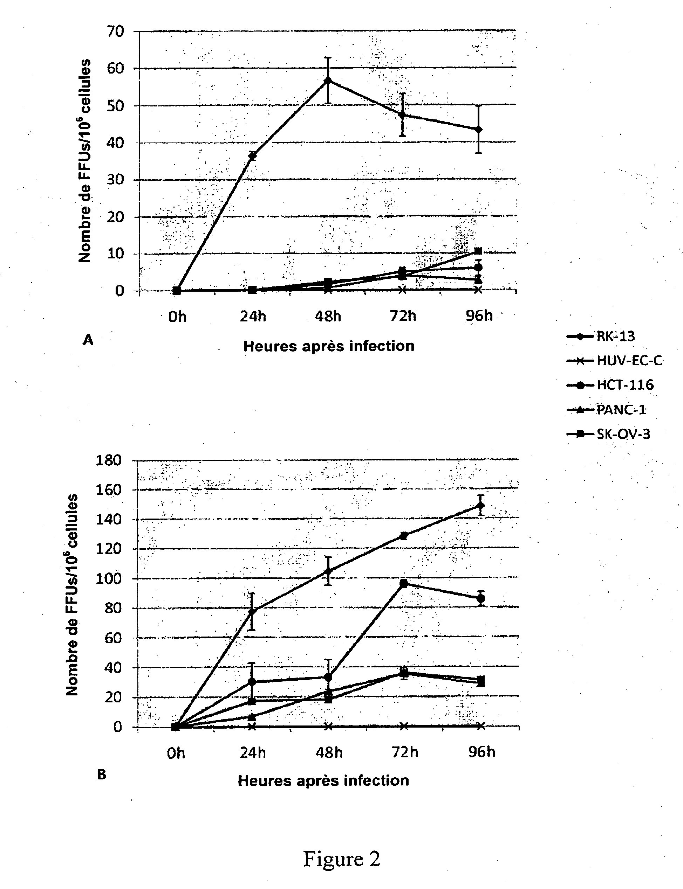

Les résultats sont illustrés par la Figure 2. (Toulouse1 :graphique A ; SG33 : graphique B). En abscisse : temps après infection ; en ordonnée : Unité Formant Foyers (FFU : Foci Forming Unit) pour 10⁶ cellules .

Ces résultats montrent que la souche SG33 diffuse plus efficacement dès 24 post-infection que la souche Toulouse 1 pour l'ensemble des cellules testées (p<0,05, test de Student).

### EXEMPLE 3 : COMPARAISON DES PROPRIETES ONCOLYTIQUES DES VIRUS TOULOUSE 1 ET SG33

La viabilité cellulaire a été mesurée par test au rouge neutre, 5 jours post-infection. Pour cela, les cellules à confluence cultivées en plaque à 96 puits ont été infectées à M.O.I de 5, et laissées à incuber pendant 5 jours. Puis, la quantité de cellules viables a été mesurée par absorption du rouge neutre (mise en contact du tapis cellulaire pendant 4 heures avec un milieu supplémenté à 3% de rouge neutre; puis lavage des cellules et incubation en présence d'alcool absolu, le relargage du rouge neutre étant suivi par mesure de la DO à 450 nm). Les résultats sont exprimés sous forme de rapport entre la moyenne des DO mesurées dans les cupules de cellules infectées et la moyenne des DO mesurées dans les cupules de cellules non infectées.

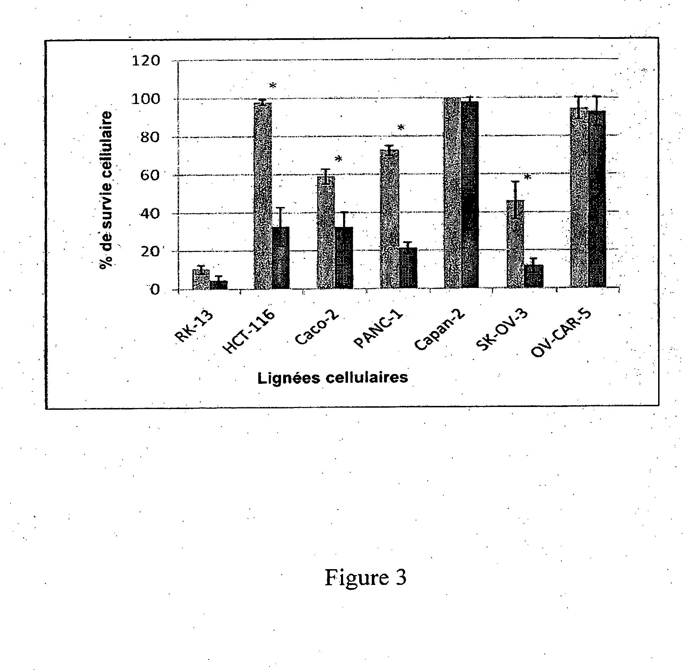

Les résultats (expérimentations réalisées en triplicat) sont illustrés par la Figure 3. Infection Toulouse 1 : barres grisées ; infection SG33 : barres noires.

Ces résultats montrent que le pourcentage de survie cellulaire est significativement différent après infection par SG33 comparée à l'infection par Toulouse 1, pour les cellules HCT-116, Caco-2, Panc-1 et SK-OV-3 (p<0,05, test de Student).

## Revendications

1. *Myxoma virus* atténué SG33 (CNCM I-1594), pour l'utilisation comme médicament oncolytique dans le traitement d'une tumeur dont les cellules sont sensibles à la lyse par ledit *myxoma virus*.

2. *Myxoma virus* atténué SG33 pour l'utilisation comme médicament oncolytique selon la revendication 1, dans le traitement d'un cancer colorectal, pancréatique ou ovarien, ou d'une tumeur gliale.

## Patentansprüche

1. Attenuiertes *Myxoma-*Virus SG33 (CNCM I-1594) zur Verwendung als onkolytisches Medikament bei der Behandlung eines Tumors, dessen Zellen für die Lyse durch das *Myxoma*-Virus empfindlich sind.

2. Attenuiertes *Myxoma*-Virus SG33 zur Verwendung als onkolytisches Medikament nach Anspruch 1 bei der Behandlung eines Kolorektal-, Pankreas- oder Eierstockkarzinoms oder eines Glioms.

## Claims

1. An attenuated *Myxoma* virus SG33 (CNCM 1-1594), for use as an oncolytic medicament for the treatment of a tumor whose cells are susceptible to lysis by said Myxoma virus.

2. SG33 *Myxoma* virus attenuated for use as oncolytic drug according to Claim 1, for the treatment of colorectal cancer, pancreatic or ovarian cancer, or glioma.
